Europäisches Patentamt

(19) European Patent Office

Office européen des brevets

(11) Publication number: **0 062 126**
**B1**

(12) **EUROPEAN PATENT SPECIFICATION**

(45) Date of publication of patent specification: **28.08.85**

(51) Int. Cl.⁴: **G 01 N 33/48**

(21) Application number: **81304838.6**

(22) Date of filing: **16.10.81**

(54) Motility fertility test.

(30) Priority: **31.03.81 US 249540**

(43) Date of publication of application:
**13.10.82 Bulletin 82/41**

(45) Publication of the grant of the patent:
**28.08.85 Bulletin 85/35**

(84) Designated Contracting States:
**DE FR GB**

(56) References cited:
**GB-A-1 230 645**

**CHEMICAL ABSTRACTS, vol. 90, no. 19, 7th
May 1979, page 261, column 2, no. 148037w,
Columbus Ohio (USA); G. YEDWAB et al.:
"Effect of caffeine on sperm penetration rate
into cervical mucus in-vitro"
CHEMICAL ABSTRACTS, vol. 88, no. 23, 5th
June 1978, page 115, column 1, no. 164622x,
Columbus Ohio (USA); W.B. SCHILL et al.:
"Improvement of cervical mucus spermatozoal
penetration by kinins - a possible therapeutical
approach in the treatment of male subfertility"**

(73) Proprietor: **SYVA COMPANY
900 Arastradero Road
Palo Alto California 94304 (US)**

(72) Inventor: **Alexander, Nancy Jeanne
17545 N.W. Autumn
Beaverton Oregon (US)**

(74) Representative: **Harrison, David Christopher
et al
MEWBURN ELLIS & CO 2/3 Cursitor Street
London EC4A 1BQ (GB)**

Courier Press, Leamington Spa, England.

## Description

The present invention relates generally to the field of human fertility testing. It is particularly concerned with a reliable reproducible method and test article for determining human sperm motility.

Measurement of sperm penetration into cervical mucus has proven to be a valuable parameter in determining the cause of infertility in couples who are unable to have children. Sperm penetration is usually measured either by examining a sample of the cervical mucus of the female collected after coitus or by examining the behavior of the sperm introduced *in vitro* into a specimen of cervical mucus.

The most widely used test of sperm penetration is the post-coital test (PCT) where the couple has intercourse and a sample of the cervical mucus is collected from two to ten hours later. Examination of the sample reveals the degree of sperm penetration and survival. Unfortunately, a lack of uniformity in the performance of the PCT makes it difficult to interpret the results. Additionally, it is impossible to distinguish between characteristics of the sperm and those of the mucus which may contribute to poor sperm penetration.

Sperm penetration is assessed *in vitro* by collecting separate samples of the semen of the male and the cervical mucus of the female. In one method, a drop of semen and a small amount of mucus are placed next to each other on a microscope slide and the sperm penetrability is subjectively determined by an investigator viewing the interaction under a microscope. It has proven difficult to standardize either the amount of mucus or semen, and variations in the size and shape of the semen-mucus interface preclude quantitative results from this form of the test.

The containment of cervical mucus in clear tubes allows a more objective measure of *in vitro* sperm penetration. A specimen of cervical mucus is collected from the female at midcycle, typically by using a catheter at the end of a syringe. The mucus is then drawn into an open-ended tube using a pipette bulb. The ends of the tube are sealed and the tube is stored until needed.

The test is performed by opening one end of the tube and placing the open end in a small amount of semen. The distance traveled by the spermatozoa within a predetermined time interval is observed under a microscope. Such a test is described in the paper entitled "Cyclic Penetrability of Human Cervical Mucus to Spermatozoa *In Vitro*" by Lamar, Shettles, and Delfs appearing in the *American Journal of Physiology*, 129:234, 1940 and the paper entitled "A Simple Sperm Penetration Test" by Kermer appearing in *The International Journal of Fertility*, 10:209, 1965.

While the use of such mucus-filled tubes has proven quite valuable, the method has several drawbacks. The use of cervical mucus drawn from the female of the infertile couple does not permit the differentiation between the separate functional characteristics of the sperm and the mucus. The use of mucus from an unrelated donor would obviate this problem, however, it would be necessary to first determine the normalcy of the donor. Furthermore, human females are not a prolific source of cervical mucus and it is sometimes difficult to obtain an adequate quantity to perform the test.

It is therefore desirable to obtain cervical mucus for use in sperm penetration testing from a source other than the human female. The source should provide large quantities of mucus, and the mucus should have a consistent quality.

An additional drawback in the prior art lies in the tendency of the mucus-filled tube to form air bubbles both at the interface with the semen sample and in the interior of the tube. Such bubbles block the progress of the sperm and can ruin test results. The mucus itself has no entrained air or other gases, and the air appears to be introduced when the mucus is drawn into the tube using the pipette bulb. Thus, it is desirable to provide a method for filling the tubes with cervical mucus which will minimize or eliminate the formation of air bubbles.

Finally, it is desirable to provide a mucus filled tube in which the microscopic structure of the micelles forming the mucus is maintained. The micelles are long protein molecules which lie generally parallel to one another while in the cervix. It has been found that disorientation of the micelles can impede sperm motility and, therefore, degrade test results.

It has been found that the mid-cycle cervical mucus of cows is similar to that of women. Laser light-scattering spectroscopy has revealed that mucus from both species consists of similar macromolecules and that the viscoelastic properties of the two are nearly identical. See the article entitled "Molecular Arrangement of Cervical Mucus: a Reevaluation Based on Laser Light-Scattering Spectroscopy" by Lee, Verdugo and Blandau in *Gynecological Investigations*, 8:254, 1977. It has further been found that human spermatozoa rapidly penetrate bovine mucus and exhibit changes in their pattern of movement identical to those displayed when penetrating human cervical mucus. See the "Letter to the Editor" by Blandau, Gaddum-Rosse and Lee in *Fertility and Sterility*, 29:707, 1978.

## Summary of the invention

A test and test article are provided which permit the rapid and accurate determination of human sperm motility in non-human cervical mucus. By preparing a mucus-filled tube in the manner of the present invention, the tendency to form air bubbles is eliminated and the microscopic structure of the mucus is maintained. Moreover, the use of bovine mucus allows for pooling of the cervical mucus to permit a large uniform source.

A tube having one open end and one sealed end is used for the test article. The open end of the tube is projected below the surface of a pool of the cervical mucus followed by subjecting it to a vacuum. By releasing the vacuum while the open end of the tube lies below the surface of the mucus, the mucus is drawn into the interior of the capillary tube. Typically, the open end of the tube is sealed and the tube is stored for future use.

Testing is carried out by breaking the tube open at one end and placing the open end into a small pool of semen. The sperm motility is ascertained by determining the rate at which the sperm penetrates axially into the tube and comparing to a normal rate.

Description of the specific embodiment
1. Preparation of mucus-filled tubes

Bovine cervical mucus or other mammalian cervical mucus having comparable properties and availability is employed. The bovine cervical mucus is obtained from estrous cows by means of rectal palpation of the cervix and extrusion of mucus to the exterior. Care should be taken to avoid fecal contamination. The mucus is collected in a container, such as a glass Petri dish. Debris in the mucus, including any white stringy material, is removed using sissors and forceps.

The tube used in the present invention must have optical qualities which allows observation of the sperm under a microscope. It is desirable to minimize the thickness of the tube to improve visibility and the length of the tube should be adequate to allow the sperm to travel a representative distance. Round capillary tubes having an internal diameter in the range from 0.5 to 1.5 mm and a length in the range from 50 mm to 100 mm have been found adequate. Flat capillary tubes are also useful, such as micro-slides #3530 available from Vitro Dynamics located in Rockaway, New Jersey, having a length in the range from approximately 50 mm to 100 mm. The cross-section of the latter capillary tubes is rectangular having internal dimensions of 3.0 mm by 0.3 mm. With both types of tube, one end must be sealed, typically by heating with a torch.

For small production of the test capillaries the following procedure may be employed. Approximately 10 ml of the cleaned bovine mucus is transferred to a common 50 ml test tube. One or more empty capillary tubes are placed with their open ends down into the test tube and the test tube is placed in a vacuum chamber. The chamber is evacuated to a vacuum of approximately 0.85 bar (25 in. Hg) for approximately 2 to 3 min. The chamber is slowly returned to atmospheric pressure over a period of from 3 to 4 min causing mucus in the test tube to enter the evacuated capillary tubes. This method of filling the tubes substantially prevents the introduction of air bubbles into the interior of the tube. Air bubbles prevent sperm from entering the tube and render a mucus-filled tube useless.

The mucus-filled tubes are then removed from the test tubes and the mucus severed using scissors. The tubes are centrifuged at 1000 rpm for 5 min in order to ensure the elimination of any air bubbles. Thereafter, the tubes are cleaned and the open end is sealed. The mucus-filled tubes may be frozen for subsequent use.

2. Test method

A semen sample is collected from the patient by masturbation after sexual abstinence of at least 48 h. A mucus-filled tube prepared in the manner described hereinabove is held at room temperature for several minutes. The tube is then scored with a diamond stylus and broken several millimeters above the mucus miniscus. The open end of the cut tube is inserted into a sample of the semen, typically 150 µl, and allowed to stand at room temperature for 90 min. The semen should interface directly with the mucus so that no air space or other obstructions prevent entry of sperm into the mucus. The capillary tube is maintained in a substantially vertical orientation during this time and the sperm attempts to penetrate upward into the mucus. The capillary tube should be carefully inspected to ensure that there is no air space or air bubbles between the fluid phases. The tube is then removed from the semen, cleaned and placed on a glass microscope slide for viewing.

The sperm typically form a phalanx and the swimming distance covered by the vanguard sperm in the phalanx is noted. Magnification of from 250 to 400× is adequate to detect the location of the sperm in the sample and a microscope having phase optics capability aids in such visual assessment.

If microscopic assessment reveals that an air bubble has blocked the tube, the sample should be discarded and the steps of the test method described above are repeated.

3. Comparative tests

Tests have been conducted comparing the results of *in vitro* sperm motility tests run with bovine mucus and with human mucus. The test results are summarized in Table One.

### TABLE ONE
## Comparison of sperm penetration in
### human and bovine cervical mucus

| Patient | Mucus penetration (mm) Human | Bovine |
|---|---|---|
| 1 | 30 | 33 |
| 2 | 21 | 20 |
| GROUP ONE 3 | 33 | 25 |
| 4 | 25 | 19 |
| 5 | 19 | 23 |
| 6 | 19 | 29 |
| 7 | 31 | 50 |
| 8 | 12 | 24 |
| 9 | 5 | 26 |
| GROUP TWO 10 | 13 | 20 |
| 11 | 4 | 21 |
| 12 | 10 | 25 |
| 13 | 0 | 35 |
| 14 | 8 | 13 |
| 15 | 13 | 10 |
| GROUP THREE 16 | 3 | 12 |
| 17 | 12 | 5 |
| 18 | 0 | 3 |

Flat capillary tubes, as described hereinabove were filled with the cervical mucus of the patient's spouse or a midcycle woman using a pipette bulb in the manner of the prior art. Additional tubes were prepared with bovine mucus in the manner of the present invention. Semen samples were collected from each patient and concurrent tests using both the human mucus-filled tubes and bovine mucus filled tubes were run. The tubes were allowed to stand in the semen samples for 90 min. at room temperature. The distance covered by the vanguard sperm was noted and the result is listed in Table One.

The patients may be divided into three groups. The patients of group one exhibited normal sperm penetration (i.e. in excess of 15 mm) in samples of their wive's cervical mucus or a midcycle woman's cervical mucus. As expected, their sperm penetration in bovine mucus was also normal. The patients of group two exhibited below normal penetration in their wive's cervical mucus, while achieving normal penetration in the bovine mucus. This result indicates either that the wive's cervical mucus is inadequate or that interaction between the particular mucus and the particular sperm prevents the sperm from penetrating. Further testing is necessary to determine the precise nature of the interaction. A third group of patients exhibited inadequate penetration in both human and bovine mucus. This indicates a deficiency in the sperm itself.

The use of bovine cervical mucus provides a useful test for determining human fertility. As evidenced by the above data no false negatives were observed in comparison with tests with human cervical mucus. As for the group which was positive with the bovine cervical mucus and negative with the human cervical mucus, without further investigation, the results cannot be interpreted. Therefore, the subject test provides substantial assurance of infertility and may require a second test for a positive result.

**Claims**

1. A method for filling a tube with cervical mucus for use in a fertility test, said tube having one open end and one sealed end, said method comprising the following steps:
   forming a pool of said cervical mucus;
   simultaneously subjecting said tube and said pool to a vacuum, whereby gas is withdrawn from the tube;
   releasing the vacuum while said end of said tube lies beneath the surface of said pool whereby the cervical mucus migrates into the interior of the tube substantially free of entrapped air.

2. A method as in Claim 1, said method comprising the further steps of:
   centrifuging the tube to expel any remaining air therefrom; and sealing the open end of the tube.

3. A method according to Claim 1 or Claim 2, wherein the cervical mucus is obtained from a bovine cow.

4. A method as claimed in any one of the preceding claims including, as a test for determining the motility of human sperm:
   introducing sperm into the cervical mucus-containing tube; and
   determining the rate at which the sperm migrates axially in the tube, as compared to sperm of known motility.

5. A mucus-filled tube free from entrapped gas bubbles for use in a test for determining human sperm motility, said tube having been prepared according to the following steps:
   simultaneously subjecting to a vacuum a pool of said cervical mucus and a tube immersed at its open end therein, whereby gas is withdrawn from the tube;
   releasing the vacuum whereby the cervical mucus passes into the interior of said tube without formation of gas bubbles therein.

**Revendications**

1. Procédé pour remplir un tube de mucus cervical en vue de son utilisation dans un test de fertilité, ledit tube ayant une extrémité ouverte et une extrémité fermée, procédé qui comprend les étapes suivantes consistant:

à rassembler un échantillon dudit mucus cervical;

à exposer simultanément ledit tube et ledit échantillon rassemblé à l'action du vide, pour l'évacuation du gaz du tube;

à casser le vide, cependent que ladite extrémité du tube se trouve au-dessous de la surface dudit échantillon rassemblé de manière que le mucus cervical émigre à l'intérieur du tube en l'absence quasi totale d'air emprisonné.

2. Procédé suivant la revendication 1, caractérisé en ce qu'il comprend les autres étapes qui consistent:

à centrifuger le tube pour en expulser tout l'air restant; et à sceller l'extrémité ouverte du tube.

3. Procédé suivant la revendication 1 ou la revendication 2, dans lequel le mucus cervical est prélevé sur une femelle de bovidé.

4. Procédé suivant l'une quelconque des revendications précédentes, consistant, à titre de test pour la détermination de la motilité du sperme humain:

à introduire du sperme dans le tube contenant du mucus cervical; et

à déterminer la vitesse à laquelle le sperme émigre axialement dans le tube comparativement à du sperme de motilité connue.

5. Tube rempli de mucus, ne contenant pas de bulles emprisonnées de gaz, destiné à être utilisé dans un test de détermination de la motilité du sperme humain, ledit tube ayant été préparé conformément aux étapes suivantes, consistant:

à soumettre simultanément à l'action d'un vide un échantillon rassemblé dudit mucus cervical et un tube qui y est immergé par son extrémité ouverte, de manière que le gaz soit évacué du tube;

à casser le vide de manière que le mucus cervical passe à l'intérieur dudit tube sans y former de bulles de gaz.

**Patentansprüche**

1. Verfahren zum Füllen eines Röhrchens, das ein offenes Ende und ein verschlossenes Ende aufweist, mit Cervixschleim zur Verwendung in einem Fertilitätstest, umfassend die folgenden Schritte:

Herstellung eines Pools von Cervixschleim;

gleichzeitiges Einwirkenlassen von Vakuum auf das Röhrchen und den Pool, wodurch Gas aus dem Röhrchen abgezogen wird;

Entspannen des Vakuums, während das Ende des Röhrchens unter der Oberfläche des Pools liegt, wodurch der Cervixschleim im wesentlichen frei von eingeschlossener Luft in das Innere des Röhrchens wandert.

2. Verfahren nach Anspruch 1, umfassend die weiteren Schritte:

Zentrifugieren des Röhrchens, um etwa zurückbleibende Luft auszutreiben, und Verschließen des oberen Endes des Röhrchens.

3. Verfahren nach Anspruch 1 oder Anspruch 2, worin der Cervixschleim von einer Rinderkuh stammt.

4. Verfahren nach irgendeinem der vorangehenden Ansprüche, umfassend als Test zur Bestimmung der Motilität von menschlischem Sperma:

Einführen von Sperma in das Cervixschleimenthaltende Röhrchen; und

Bestimmen der Geschwindigkeit, mit der das Sperma axial in dem Röhrchen wandert im Vergleich zu Sperma von bekannter Motilität.

5. Mit Schleim gefülltes Röhrchen, das frei ist von eingeschlossenen Gasblasen, zur Verwendung in einem Test zur Bestimmung der Motilität von menschlichem Sperma, welches Röhrchen hergestellt worden ist in folgenden Schritten:

Gleichzeitiges Einwirkenlassen von Vakuum auf einen Pool von Cervixschleim und ein Röhrchen, das mit seinem offenen Ende darin eintaucht, wodurch Gas aus dem Röhrchen abgezogen wird;

Entspannen des Vakuums, wodurch der Cervixschleim in das Innere des Röhrchens eintritt, ohne daß sich darin Gasblasen bilden.